Europäisches Patentamt

European Patent Office

Office européen des brevets

(11) Publication number: **0 109 059**
**B1**

# (12) EUROPEAN PATENT SPECIFICATION

(45) Date of publication of patent specification: **15.07.87**

(21) Application number: **83111240.4**

(22) Date of filing: **10.11.83**

(51) Int. Cl.[4]: **C 07 C 11/06,** C 07 C 4/10,
B 01 J 29/28, B 01 J 29/38

(54) **Process for converting olefins having 4 to 12 carbon atoms into propylene.**

(30) Priority: **10.11.82 IT 2415282**
**02.12.82 IT 2455082**
**27.01.83 IT 1929283**
**03.05.83 IT 2089683**

(43) Date of publication of application:
**23.05.84 Bulletin 84/21**

(45) Publication of the grant of the patent:
**15.07.87 Bulletin 87/29**

(84) Designated Contracting States:
**DE FR GB IT**

(56) References cited:
**EP-A-0 036 704**
**EP-A-0 037 671**
**US-A-4 150 062**

(73) Proprietor: **MONTEDIPE S.p.A.**
**31, Foro Buonaparte**
**Milan (IT)**

(72) Inventor: **Colombo, Fausto**
**60/A, Via Verdi**
**Merate (Como) (IT)**
Inventor: **Alberti, de Giordano**
**4, Largo Brianzoni**
**Besnate (Varese) (IT)**
Inventor: **Moretti, Enrico**
**3/G, Largo Ungaretti**
**Arese (Milano) (IT)**
Inventor: **Paparatto, Guiseppe**
**8, Via Buozzi**
**Cinisello Balsamo (Milano) (IT)**
Inventor: **Contessa, Socrate**
**35, Via Repubblica**
**Senago (Milano) (IT)**

(74) Representative: **Barz, Peter, Dr. et al**
**Patentanwälte Dr. V. Schmied-Kowarzik Dipl.-**
**Ing. G. Dannenberg Dr. P. Weinhold Dr. D. Gudel**
**Dipl.-Ing. S. Schubert Dr. P. Barz**
**Siegfriedstrasse 8**
**D-8000 München 40 (DE)**

**Description**

The invention relates to a process for converting olefins having 4 to 12 carbon atoms into propylene.

Nowadays, huge amounts of olefinic cuts from $C_4^-$ to $C_{12}^-$, either linear or branched, are available throughout the world and they are widely employed for different purposes, such as described e.g. in Italian patent publications 24152 A/82, 24550 A/82 and 19292 A/83.

Sometimes, however, because of contingent reasons even outside the chemical field such as, for instance, transportation difficulties, it would be better to have still further possibilities of use.

A promising use of said olefins would be their conversion into propylene and/or ethylene.

EP—A—37 671 discloses a process for converting large olefins into smaller olefins by contacting said larger olefins with e.g. ZSM5 or ZSM11 zeolites having a $SiO_2/Al_2O_3$ molar ratio of at least 12, at a temperature of e.g. 400 to 600°C, whereby said zeolites are used as such or in a modified form and preferably have a particle size of 0.5 to 100 µm. This process is, however, carried out at rather low space velocities and thus results in a limited output of the desired smaller olefins in a very low yield.

It is an object of the present invention to provide a process for converting olefinic cuts from $C_4^-$ to $C_{12}^-$ to propylene and, optionally, also ethylene with a high yield and output of these olefins.

In its most general form, the invention relates to a process for converting olefins having from 4 to 12 carbon atoms into propylene by contacting said olefins at a temperature of from 400 to 600°C with a ZSM5 or ZSM11 zeolite having a $SiO_2/Al_2O_3$ molar ratio (Z ratio)≤300 which is used as such or in a modified form, optionally admixed with an inert binder, which is characterized in that the zeolite crystallites have an average size of ≤0.5 µm and that the conversion is carried out at a space velocity higher than 50 kg/h of olefins per kg of pure zeolite (binder excluded).

Generally, the lower the Z ratio, the higher the space velocity; the examples show very good results corresponding to a Z ratio of 28, but higher ratios are equally or more effective.

The catalyst comprises preferably zeolites of the ZSM type, as described e.g. in European patent publication 36707; best results were attained by using ZSM 5 or ZSM 11 zeolites in their acid form (H—ZSM 5 or H—ZSM 11), or in a modified form (for instance by means of phosphorus, magnesium, calcium, strontium, barium, chromium, copper or zinc).

The modifying element can be incorporated into the catalyst by means of ion exchange or by other methods, for example impregnation or co-precipitation during the zeolite synthesis.

According to a particularly advantageous way for the preparation of a non-modified catalyst (e.g. H—ZSM 5), the raw product coming from the zeolite synthesis is dried, for instance at 120°C, calcined (e.g. at 540°C for some hours, in order to displace all the residual templating agent) and then exchanged with an aqueous solution of HCl, $NH_4Cl$, $NH_4NO_3$ or an equivalent $H^+$ or $NH_4^+$ source. When an ammonium compound is used, it is necessary to heat, for instance at 400°C, in order to obtain the acid form. A survey of alternative techniques for the ion exchange is given e.g. in US patents 3,140,249; 3,140,251; 3,140,253 and in European patent publications 30796, 36707, 37168, 40463 and 68754. The zeolite, after calcination and conversion into the acid form, has a long life and a very high catalytic activity. The zeolites mentioned above can be used as catalysts (as such or in a modified form) optionally in admixture with suitable amounts of oxides as binders, for instance $SiO_2$ or $Al_2O_3$. Other binders are disclosed e.g. in European patent publication 36707.

The regeneration of the catalyst can be carried out in air for some hours at 400—600°C. A steam regeneration is described in European patent publication 36704 and according to a further and very successful method, the catalyst can be regenerated by a hydrogen treatment.

As to the initial activation of the catalyst, some methods are described in European patent publications 34444 and 35830; in general, it is advisable to activate the catalyst for some hours in air at 450—750°C (preferably at 540—700°C).

Any process for the conversion of olefinic cuts to propylene will be indicated hereafter as a "post-pyrolysis" process. When the raw material of a post-pyrolysis process is a mixture of olefins having 4C atoms, there is a considerable problem to be solved, because the $C_4^-$ cuts always contain substantial amounts of paraffins in general also having 4C atoms, which paraffins pass (at 400—600°C) the zeolite bed without taking part in any reaction.

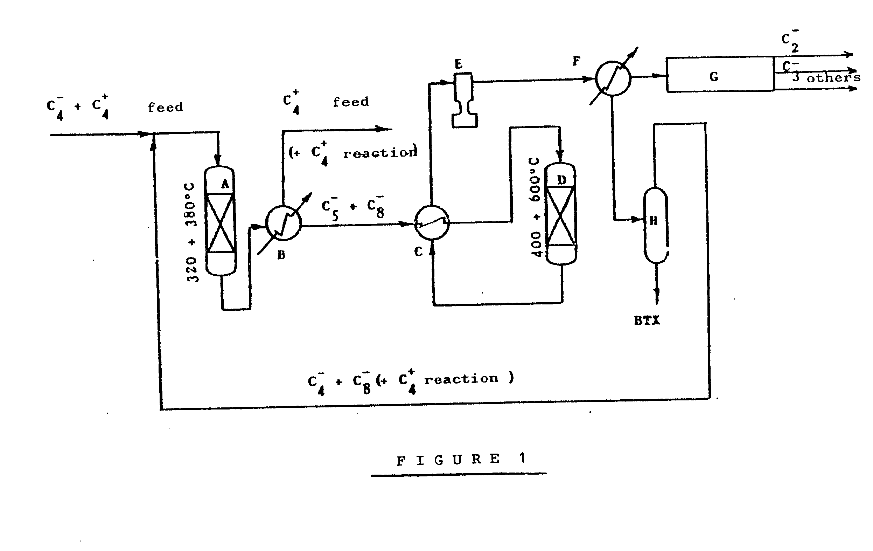

Furthermore, a small amount of $C_4$ paraffins is produced by the post-pyrolysis process itself. The conversion to $C_3H_6$ could be enhanced by a recycle of the non-reacted $C_4^-$ olefins or of the $C_4^-$ olefins formed during the reaction. In such a case however, an increasing accumulation of n-butane and of isobutane would take place. This drawback could be avoided by separating paraffins from olefins before feeding the reactor, but such a separation is rather difficult. Butenes and isobutane cannot be isolated by a simple distillation and it is usually necessary to carry out an extractive distillation (a troublesome technique) which is particularly difficult for the $C_4^-$ cuts coming from catalytic cracking, where butane and isobutane may account for up to 50% of the whole. The problem however can be solved in a surprisingly easy way by the process according to the invention, when employing an integrated oligomerization step. In other words, a particular embodiment of the invention (the feed being a paraffin-olefin mixture) comprises the following steps (reference is made also to figure 1):

a) a preliminary oligomerization of a $(C_4^- + C_4^+)$ mixture at a temperature of from 320 to 380°C, using a

2

catalyst bed of zeolitic nature (see e.g. US patent No. 4,150,062) to obtain a mixture of olefins having from 5 to 8C atoms, the $C_4^+$ paraffins remaining substantially unconverted;

b) cooling and condensation of the oligomerization effluent in order to separate the $C_4^+$ paraffins as a gaseous phase, and conversion of the remaining $(C_5^- - C_8^-)$ mixture to propylene under the typical post-pyrolysis reaction conditions described above;

c) cooling the effluent from the reactor for the conversion to propylene and compression of said effluent, preferably at 13—16 bar, whereby the hydrocarbons having 4 or more C atoms are condensed and the hydrocarbons having less than 4C atoms are separated as a gaseous phase.

These hydrocarbons $(<C_4)$ can be advantageously fed to a conventional battery of distillation columns for thermal or catalytic cracking in order to recover all the propylene contained therein. The small amounts of aromatics (BTX) can be easily separated from the other $\geqslant C_4$ hydrocarbons, if necessary, and recycled together with the final $(C_4^- - C_8^-)$ mixture, containing small amounts of butanes, produced during the conversion reaction described under item (b) above. In order to carry out the oligomerization, $C_4^-$ olefins containing $C_4^+$ paraffins in any proportion, are initially brought into contact with a catalyst of zeolitic nature, for instance ZSM 5 or ZSM 11, in an acid or in a modified form, at 250—400°C (preferably 320—380°C) and at space velocities from 2 to 10 (preferably 4—8) kg/h of reactants per kg of pure zeolite (binder excluded). In other words, the olefins of the $(C_4^- + C_4^+)$ mixture are converted almost totally into a $(C_5^- - C_8^-)$ olefinic mixture, while butane and isobutane do not react. The separation of the butanes can thus be carried out very easily by simple cooling with water at room temperature. The olefinic $(C_5^- - C_8^-)$ mixture liquifies while the butanes are separated as a gaseous phase, said $(C_5^- - C_8^-)$ mixtures being optimal raw materials for the production of propylene. Depending on the working conditions during the synthesis of the zeolite, such as e.g. the dilution of the starting solutions, the zeolite crystallites may have a widely variable size; in general, a sharp subdivision is noted at 0.5—1 µm, i.e. the crystallites' dimensions are generally above that level ("large size" crystals) or below that level ("small size" crystals). The catalysts according to the present invention are "small size" crystals and can e.g. be prepared according to the methods of US patent No. 3,926,782.

The following examples are given merely for illustrative purposes and do not in any way limit the scope of the invention.

Working conditions common to all the examples
a) Zeolite ZSM 5 was prepared according to example 24 of US patent 3,702,886 with stirring, so as to obtain crystallites having an average size below 0.5 µm;
b) zeolite ZSM 11 was prepared according to US patent 3,709,979 with stirring, so as to obtain crystallites having an average size below 0.5 µm;
c) the modified zeolites were obtained by ion exchange, unless otherwise specified in the tables;
d) all the catalysts were activated prior to use at 540°C for 2 hours, unless otherwise specified in the tables.

Examples 1 to 20
The zeolites, mixed with $SiO_2$ as a binder, were charged into a microreactor which was continuously fed at a pressure slightly higher than atmospheric pressure with a mixture consisting of equal proportions (1:1) of 2-cis-butene and 2-trans-butene. The reaction conditions and results are given in Table 1.

Example 21 (Regeneration Test)
After an 18-hour run according to Example 20, the reaction was stopped and the catalyst was regenerated with air, at atmospheric pressure and at the reaction temperature (550°C) for 2 hours; feeding of butenes was started again according to the conditions of Example 25 and after 1 hour the following results were obtained:

| | |
|---|---|
| Conversion | 88.60% |
| Selectivity to iso $C_4^-$ | 8.62% |
| Selectivity to $C_3^-$ | 38.50% |
| Yield of iso $C_4^- + C_3^-$ | 41.75% |
| Selectivity to $\geqslant C_5$ | 33.54% |
| Selectivity to saturated gases $\leqslant C_4$ | 11.97% |
| Selectivity to $C_2^-$ | 7.37% |

Examples 22 to 44
0.5 g of zeolite, mixed with 0.21 g of $Al_2O_3$ as a binder, were charged into a microreactor which was continuously fed, at a pressure slightly higher than atmospheric pressure with an 0.7:1 (by moles) mixture of n-pentene-1 and helium. The reaction conditions and results are given in Table 2.

Examples 45 to 48
These Examples were carried out according to the preceding Examples, but pentene was substituted by n-hexene (Examples 45 to 46) and n-octene-1 (Examples 47 and 48), respectively. Data and results are given in Table 3.

Examples 49 and 50

These Examples were carried out according to Examples 22 to 44, but pentene was substituted by an olefin fraction C$_5$, coming from a fluid bed catalytic cracking (FCC) and essentially consisting of hydrocarbons having 5 carbon atoms with approximately the following composition:

Olefins (C$_5^-$)=70% by weight;
Paraffins (C$_5^+$)=30% by weight;
Dienes≤0.5 by weight.
Data and results are given in Table 3.

Examples 51 and 52

Examples 45 and 46 were repeated, substituting linear hexene by a branched isomer (2-methyl-1-pentene). Data and results are given in Table 3.

It is apparent from the above that the cracking of branched olefins according to the present invention substantially leads to the same results as the cracking of the corresponding linear olefins. This is surprising in so far as it was believed until now (see European patent publication 37,671) that linear olefins were far easier to convert in the presence of zeolites than the branched isomers.

Examples 53 to 60

3 g of H—ZSM5 mixed with 0.9 g of SiO$_2$ as a binder, were loaded into a microreactor which was continuously fed with isobutene, at a pressure slightly higher than atmospheric pressure. Reaction conditions and results are given in Table 4.

These tests show the influence of the temperature on the reaction.

Examples 61 to 68

These tests were carried out by repeating Example 55 (at 550°C) and varying the space velocity, i.e. the residence time. In this way it was possible to ascertain that for velocities up to about 311 h$^{-1}$, the increase in selectivity to C$_3^-$ compensates at least partially the decrease suffered by the conversion in the long run. Data and results are indicated in Table 4.

Examples 69 and 70

These tests were carried out by repeating Example 65 and raising the temperature to 580°C. Data and the excellent results are indicated in Table 4.

Examples 71 and 72

These tests were carried out by repeating Example 55, employing a zeolite H—ZSM5 with a much higher SiO$_2$/Al$_2$O$_3$ molar ratio (204). The data and results given in Table 4 show that the propylene yield was positively influenced by the increase in said ratio. However, if such ratio increases excessively, a drastic reduction in the C$_3^-$ yield occurs at higher space velocities.

Examples 73 and 74

These tests were performed by repeating Example 57 and employing a zeolite activated for 2 hours at a higher temperature i.e. at 700°C; the reaction data are indicated in Table 4 together with the excellent results which prove the positive influence exerted by the increase in the catalyst activation temperature.

Examples 75 and 76

These tests were carried out by repeating Example 57 and substituting the zeolite in the acid form by a zeolite partially exchanged with Cr; the exchanged zeolite contained 0.8% by weight of Cr. Data and results are in Table 4.

Examples 77 to 79

These tests were performed by repeating Example 57 and substituting the zeolite in the acid form by a zeolite exchanged with Mg. The tests prove that a modification with Mg exhibits excellent aspects (see Table 4).

Examples 80 and 81 (Comparative Tests)

These tests were performed by repeating Example 55 and reducing the space velocity to a very low value (3.5 h$^{-1}$). The low propylene yield clearly proves the importance of a velocity exceeding the critical value (50 h$^{-1}$); see Table 4.

Example 82
Part A

As shown in Figure 1, a reactor (A) was loaded with 10 g of the H—ZSM5 of Example 1 and was then continuously fed, at 2 bar and 350°C, with the 50/50 butenes mixture of Example 1, the space velocity being 12.5 kg/h of butenes per kg of H—ZSM5; the gaseous products were cooled and condensed (at 15°C), thus obtaining 61.1 g/h of liquids and 63.9 g/h of gas. The gases contained 95.6% by weight of isobutane, the

4

rest being light olefins and paraffins ($\leq C_4$) and the liquids contained 99% by weight of a ($C_5{}^- - C_8{}^-$) mixture, the rest being aromatics.

Part B

The liquids obtained according to Part A were fed continuously to a second reactor (D) at 550°C and atmospheric pressure, the space velocity being WHSV=65 h$^{-1}$; said reactor had been loaded with 3.3 g of the same H—ZSM5 of Part A. After a 4-hour run, the effluent from reactor (D) had the following composition (% by weight):

| | | | |
|---|---|---|---|
| $C_2{}^+$ | 0,39 | iso-$C_4{}^-$ | 8,50 |
| $C_2{}^-$ | 4,87 | $C_5{}^- + C_6{}^-$ | 18,53 |
| $C_3{}^+$ | 6,44 | Benzene | 1,06 |
| $C_3{}^-$ | 31,88 | Toluene | 5,28 |
| $C_4{}^+$ | 4,06 | Xylenes | 6,16 |
| n-$C_4{}^-$ | 12,83 | | |

(Benzene, Toluene, Xylenes = BTX)

Said effluent was used for the pre-heating of the feed stream (of reactor D) and was then compressed by (E) and cooled with condenser (F), resulting in:

a) a gaseous stream, rich in propylene, which can be rectified in (G), so as to recover $C_3{}^-$ and, optionally also $C_2{}^-$;

b) a liquid stream, which can also be rectified—in (H)—so as to separate, if necessary, the very small amount of aromatics.

Example 83

Part B of Example 82 was repeated, increasing the space velocity to 128 h$^{-1}$, after a 4-hour run the effluent from (D) had the following composition (% by weight):

| | | | |
|---|---|---|---|
| $C_2{}^+$ | 0,34 | iso-$C_4{}^-$ | 7,39 |
| $C_2{}^-$ | 5,54 | $C_5{}^- + C_6{}^-$ | 25,85 |
| $C_3{}^+$ | 5,15 | Benzene | 1,03 |
| $C_3{}^-$ | 32,37 | Toluene | 4,72 |
| $C_4{}^+$ | 2,99 | Xylenes | 5,12 |
| n-$C_4{}^-$ | 9,50 | | |

(Benzene, Toluene, Xylenes = BTX)

TABLE 1

| Example | 1 | 2 | 3 | 4 | 5 | 6 | 7 |
|---|---|---|---|---|---|---|---|
| Operative conditions: Catalyst | H—ZSM5(b) | See ex. 1 | See ex. 1 | H—ZSM5 | See ex. 4 | H—ZSM11 | See ex. 6 |
| $SiO_2/Al_2O_3$ (moles) | 28 | " | " | 28 | " | 82 | " |
| Amount of catalyst | (c) | " | " | 3 grams (d) | " | see ex. 4 | " |
| Temp. (°C) | 500 | " | " | 400 | 550 | 500 | " |
| Space velocity (a) | 60 | 215 | 427 | 60 | 60 | 60 | " |
| Data survey after: | 6h | 6h | 6h | 6h | 6h | 1h | 6h |
| Results (%b.w.) Conversion | 88.58 | 80.05 | 74.55 | 91.11 | 87.64 | 88.45 | 86.03 |
| Selectivity to iso-$C_4^-$ | 9.28 | 15.26 | 17.37 | 7.58 | 9.39 | 9.46 | 11.60 |
| Selectivity to $C_3^-$ | 31.99 | 34.32 | 34.37 | 16.74 | 40.65 | 33.10 | 35.03 |
| Yield (iso $C_4^-$+$C_3^-$) | 36.56 | 39.69 | 38.57 | 21.25 | 43.86 | 37.64 | 40.12 |
| Sel. to compounds $\geq C_5$ | 41.31 | 42.60 | 42.22 | 69.15 | 31.80 | 41.10 | 41.55 |
| Sel. to saturated compounds $\leq C_4$ | 13.19 | 5.64 | 4.32 | 5.52 | 10.80 | 11.95 | 8.09 |
| Selectivity to $C_2^-$ | 4.22 | 2.28 | 1.73 | 1.02 | 7.37 | 4.39 | 3.73 |

(a) WHSV (Weight Hourly Space Velocity) namely Kg/h of olefines per Kg. of pure zeolite (binder excluded);
(b) +30% b.w. $SiO_2$ (binder);
(c) depending on pre-fixed WHSV;
(d) 3 g of Zeolite+0.9 g of $SiO_2$.

TABLE 1 (Continuation)

| Example | 8 | 9 | 10 | 11 | 12 | 13 |
|---|---|---|---|---|---|---|
| | | | | | (**) | (**) |
| Operative conditions:<br>Catalyst | H—ZSM5 | See ex 8 | H—ZSM5 | H—P—ZSM5 | H—ZSM5 | See ex. 12 |
| $SiO_2/Al_2O_3$ (moles) | 204 | " | 28 | see ex. 10 | 28 | " |
| Amount of catalyst | see ex. 4 | " | see ex. 4 | " | see ex. 4 | " |
| Temp. (°C) | 550 | " | 500 | " | 550 | " |
| Space velocity (a) | 60 | " | 60 | " | 82 | " |
| Data survey after: | 1h | 6h | 1h | " | 1h | 6h |
| Results (%b.w.)<br>Conversion | 88.02 | 84.26 | 93.11 | 80.97 | 86.42 | 83.33 |
| Selectivity to iso-$C_4^-$ | 9.13 | 12.52 | 5.44 | 14.97 | 10.41 | 13.02 |
| Selectivity to $C_3^-$ | 37.98 | 38.01 | 28.14 | 36.68 | 44.36 | 45.72 |
| Yield (iso $C_4^-$+$C_3^-$) | 41.55 | 42.50 | 31.27 | 41.82 | 47.33 | 49.00 |
| Sel. to compounds $\geqslant C_5$ | 35.77 | 39.32 | 40.54 | 40.48 | 27.48 | 27.87 |
| Sel. to saturated<br>compounds $\leqslant C_4$ | 10.36 | 5.54 | 21.34 | 5.22 | 10.34 | 7.49 |
| Selectivity to $C_2^-$ | 6.75 | 4.71 | 4.54 | 2.66 | 7.41 | 5.85 |

(a) WHSV (Weight Hourly Space Velocity) namely Kg/h of olefines per Kg. of pure zeolite (binder excluded);

(*) Zeolite H—P—ZSM5 was containing 3.17 b.w. of phosphorus; trimethyl-phosphite was added according to Vedrine et. al. "J. Catal. 73, 147 (1982).

(**) Zeolite H—ZSM5 was activated 2h at 700°C in air, before being used.

0 109 059

TABLE 1 (Continuation)

| Example | 14 | 15 | 16 | 17 | 18 | 19 | 20 |
|---|---|---|---|---|---|---|---|
| Operative conditions: Catalyst | Zn—ZSM5 | Cr—ZSM5 | See ex. 15 | Cu—ZSM5 | See ex. 17 | See ex. 17 | See ex. 19 |
| $SiO_2/Al_2O_3$ (moles) | 28 | See ex. 14 | " | See ex. 14 | " | " | " |
| Amount of catalyst | See ex. 4 | " | " | " | " | " | " |
| Temp. (°C) | 550 | " | " | " | " | " | " |
| Space velocity (a) | 60 | " | " | " | " | 83 | " |
| Data survey after: | 6h | 1h | 6h | 1h | 6h | 1h | 6h |
| Results (%b.w.) Conversion | 90.67 | 89.86 | 88.27 | 93.66 | 80.99 | 88.62 | 79.30 |
| Selectivity to iso-$C_4^-$ | 6.88 | 7.50 | 8.88 | 4.45 | 15.56 | 8.74 | 16.86 |
| Selectivity to $C_3^-$ | 32.10 | 37.34 | 38.57 | 26.48 | 43.42 | 38.83 | 42.13 |
| Yield (iso $C_4^-$+$C_3^-$) | 35.34 | 40.29 | 41.88 | 28.97 | 47.77 | 42.12 | 46.78 |
| Sel. to compounds $\geqslant C_5$ | 46.62 | 34.39 | 35.81 | 47.94 | 28.73 | 34.70 | 30.41 |
| Sel. to saturated compounds $\leqslant C_4$ | 8.49 | 12.81 | 9.47 | 14.35 | 7.09 | 10.35 | 5.79 |
| Selectivity to $C_2^-$ | 5.92 | 7.97 | 7.26 | 6.79 | 5.19 | 7.40 | 4.81 |

(a) WHSV (Weight Hourly Space Velocity) namely Kg/h of olefines per Kg. of pure zeolite (binder excluded):

TABLE 2

| Example | 22 | 23 | 24 | 25 | 26 | 27 | 28 | 29 |
|---|---|---|---|---|---|---|---|---|
| Operative Conditions:<br>Catalyst | H—ZSM5 | H—ZSM5 | See ex. 23 | See ex. 23 | See ex. 25 | See ex. 23 | See ex. 27 | See ex. 23 |
| $SiO_2/Al_2O_3$ (moles) | 26 | 28 | " | " | " | " | " | " |
| Amount of catalyst | (c) | (c) | " | " | " | " | " | " |
| Temp. (°C) | 400 | 500 | " | " | " | 550 | " | 585 |
| Space velocity (a) | 71.4 | 71.4 | " | 142.8 | " | 71.4 | " | 178.6 |
| Data survey after: | 1h | 1h | 6h | 1h | 6h | 1h | 6h | 7h |
| Results (% b.w.)<br>Conversion | 65.31 | 82.60 | 78.92 | 70.40 | 62.88 | 88.98 | 84.25 | 82.83 |
| Selectivity to $C_2^-$ | 4.01 | 13.50 | 13.46 | 12.77 | 12.45 | 21.26 | 21.87 | 25.32 |
| Selectivity to $C_3^-$ | 23.30 | 27.36 | 33.06 | 33.71 | 36.50 | 30.31 | 37.50 | 36.69 |
| Yield $(C_2^- + C_3^-)$ | 17.83 | 33.75 | 36.71 | 32.72 | 30.78 | 45.89 | 49.99 | 51.36 |
| Sel. to saturated<br>compounds $<C_4$ | 2.94 | 8.20 | 5.04 | 4.33 | 2.77 | 6.46 | 3.11 | 2.36 |
| Sel. to $C_4^-$ | 30.84 | 17.37 | 23.62 | 27.44 | 32.16 | 13.28 | 19.54 | 17.67 |
| Sel. to $C_4^+$ | 12.00 | 12.41 | 8.78 | 7.36 | 5.23 | 6.81 | 4.01 | 2.47 |
| Sel. to BTX (b) | 10.14 | 15.84 | 10.66 | 9.26 | 6.10 | 19.07 | 11.27 | 12.83 |
| Sel. to other<br>liquids | 17.27 | 5.20 | 5.22 | 5.31 | 4.90 | 2.79 | 2.60 | 1.93 |

(a) See ex. 1; (b) BTX=benzene+toluene+xylenes; (c) 0.5 g of pure zeolite+0.21 g of $Al_2O_3$ (binder).

TABLE 2 (Continuation)

| Example | 30 | 31 | 32 | 33 | 34 | 35 | 36 |
|---|---|---|---|---|---|---|---|
| Operative Conditions: Catalyst | (c) H—ZSM5 | (c) See ex. 30 | (d) H—ZSM5 | (*) Cr—H—ZSM5 | (*) See ex. 33 | (**) Zn—ZSM5 | (**) See ex. 35 |
| $SiO_2/Al_2O_3$ (moles) | 28 | " | see ex. 30 | see ex. 30 | " | 28 | " |
| Amount of catalyst | see ex. 23 | " | " | " | " | see ex. 23 | " |
| Temp. (°C) | 500 | " | " | " | " | 550 | " |
| Space velocity (a) | 55.7 | " | " | " | " | 71.4 | " |
| Data survey after: | 1h | 6h | 6h | ?h | 5.25h | 1h | 6h |
| Results (% b.w.) Conversion | 81.08 | 77.69 | 65.78 | 77.55 | 75.89 | 89.10 | 81.10 |
| Selectivity to $C_2^-$ | 12.56 | 12.71 | 11.28 | 12.70 | 12.49 | 20.71 | 21.69 |
| Selectivity to $C_3^-$ | 28.71 | 32.85 | 34.93 | 32.04 | 34.77 | 25.02 | 29.89 |
| Yield $(C_2^-+C_3^-)$ | 33.46 | 35.40 | 30.40 | 34.70 | 35.87 | 40.75 | 41.83 |
| Sel. to saturated compounds $<C_4$ | 7.44 | 5.24 | 3.30 | 4.80 | 4.14 | 1.73 | 1.01 |
| Sel. to $C_4^-$ | 19.31 | 24.86 | 34.14 | 25.07 | 25.77 | 11.66 | 15.65 |
| Sel. to $C_4^+$ | 11.93 | 9.18 | 4.74 | 9.35 | 7.35 | 4.20 | 2.28 |
| Sel. to BTX (b) | 13.80 | 11.08 | 4.82 | 12.02 | 9.75 | 32.92 | 23.30 |
| Sel to other liquids | 6.17 | 6.39 | 5.94 | 6.00 | 5.73 | 3.64 | 2.81 |

(a) See ex. 1; (b) BTX=benzene+toluene+xylenes; (c) Zeolite H—ZSM5 was activated 2h at 600°C before being used;
(d) Zeolite H—ZSM5 was activated 2h at 700°C;
(*) Chromium was added by means of a partial ion exchange.
(**) Zn was added by means of a complete ion exchange, as to occupy practically all the sites within the Zeolite.

TABLE 2 (Continuation)

| Example | 37 | 38 | 39 | 40 | 41 | 42 | 43 | 44 |
|---|---|---|---|---|---|---|---|---|
| Operative Conditions: Catalyst | (**) Cr—ZSM5 | (**) See ex. 37 | (**) Cu—ZSM5 | (**) See ex. 39 | H—ZSM11 | (***) Na—H—ZSM11 | H—ZSM5 | (****) P—H—ZSM5 |
| $SiO_2/Al_2O_3$ (moles) | See ex. 35 | " | See ex. 35 | " | 80 | See ex. 41 | 208 | 28 |
| Amount of catalyst | " | " | " | " | See ex. 23 | " | See ex. 41 | See ex. 41 |
| Temp. (°C) | " | " | " | " | 550 | " | " | " |
| Space velocity (a) | " | " | " | " | 71.4 | " | " | " |
| Data survey after: | 1h | 6h | 1h | 6h | 6h | 6h | 6h | 6h |
| Results (% b.w.) Conversion | 84.75 | 77.96 | 89.56 | 78.33 | 63.84 | 63.06 | 49.43 | 70.56 |
| Selectivity to $C_2^-$ | 21.85 | 21.66 | 20.69 | 22.42 | 20.34 | 20.07 | 21.02 | 22.05 |
| Selectivity to $C_3^-$ | 37.65 | 38.48 | 28.06 | 40.48 | 40.22 | 40.27 | 41.29 | 38.36 |
| Yield $(C_2^- + C_3^-)$ | 50.43 | 46.89 | 43.66 | 49.27 | 38.39 | 38.05 | 30.80 | 42.63 |
| Sel. to saturated compounds $<C_4$ | 3.47 | 3.46 | 4.36 | 2.48 | 1.28 | 0.96 | 0.77 | 2.84 |
| Sel. to $C_4^-$ | 19.39 | 24.86 | 13.58 | 23.82 | 29.17 | 30.16 | 29.96 | 24.91 |
| Sel. to $C_4^+$ | 4.01 | 2.28 | 4.00 | 1.90 | 1.74 | 1.20 | 0.89 | 2.86 |
| Sel. to BTX (b) | 10.98 | 7.87 | 25.69 | 6.34 | 4.60 | 4.41 | 3.42 | 7.22 |
| Sel to other liquids | 2.60 | 3.18 | 4.30 | 2.56 | 2.61 | 2.92 | 2.61 | 1.71 |

(a) See ex. 1 (b) BTX=benzene+toluene+xylenes.
(**) See ex. 35;
(***) Na occupies 50% of the sites within the zeolite;
(****) See ex. 11.

0 109 059

TABLE 3

| Example | 45 | 46 | 47 | 48 | 49 | 50 | 51 | 52 |
|---|---|---|---|---|---|---|---|---|
| Operative Conditions: Catalyst | (*) H—ZSM5 | (*) See ex. 45 | (**) See ex. 45 | (**) See ex. 45 | (***) See ex. 45 | (***) See ex. 49 | (****) H—ZSM5 | (*****) See ex. 51 |
| $SiO_2/Al_2O_3$ (moles) | 28 | '' | '' | '' | 28 | '' | 28 | '' |
| Amount of catalyst | see ex. 23 | '' | '' | '' | see ex. 23 | '' | see ex. 23 | '' |
| Temp. (°C) | 550 | '' | '' | '' | 585 | '' | 550 | '' |
| Space velocity (a) | 71.4 | '' | '' | '' | 178.5 | 214 | 71.4 | 357 |
| Data survey after: | 1h | 6h | 1h | 6h | 7h | 1h | 6h | 7h |
| Results (% b.w.) Conversion | 97.46 | 96.90 | 100 | 100 | 54.81 | 64.85 | 100 | 100 |
| Selectivity to $C_2^=$ | 14.49 | 13.14 | 16.97 | 14.27 | 21.13 | 24.09 | 12.49 | 14.49 |
| Selectivity to $C_3^=$ | 31.18 | 45.14 | 27.33 | 32.81 | 41.63 | 36.70 | 41.96 | 41.84 |
| Yield $(C_2^=+C_3^=)$ | 44.51 | 56.47 | 44.30 | 47.08 | 34.40 | 39.42 | 54.45 | 56.33 |
| Sel. to saturated compounds $<C_4$ | 6.54 | 2.12 | 6.00 | 2.93 | 1.97 | 3.72 | 1.86 | 1.01 |
| Sel. to $C_4^=$ | 15.99 | 19.71 | 12.62 | 18.04 | 18.21 | 15.28 | 17.96 | 18.04 |
| Sel. to $C_4^+$ | 5.84 | 1.43 | 7.13 | 4.02 | 1.53 | 3.19 | 1.28 | 0.58 |
| el. to BTX (b) | 11.81 | 3.42 | 16.13 | 10.83 | 10.29 | 13.26 | 6.97 | 6.14 |
| Sel. to $C_5$ | 10.42 | 12.81 | 8.66 | 11.06 | — | — | 14.80 | 14.18 |
| Sel. to others | 3.68 | 2.26 | 5.13 | 5.67 | 5.20 | 3.90 | 2.68 | 3.72 |

(a) See ex. 1 (b) BTX=benzene+toluene+xylenes; (*) Feed=n-hexene-1; (**) Feed=n-octene-1; (***) Feed=$C_5$ cut from F.C.C., containing $C_5^+$, $C_5^-$ and dienes; (****) Feed=2-methyl-pentene+He; olefine/He=0.7:1 by moles; (*****) Feed=pure 2-methyl-pentene, without He as diluent.

0 109 059

TABLE 4

| Example | 53 | 54 | 55 | 56 | 57 | 58 | 59 | 60 |
|---|---|---|---|---|---|---|---|---|
| Operative Conditions: Catalyst | H—ZSM5 | See ex. 53 | See ex. 53 | See ex. 55 | See ex. 53 | See ex. 57 | See ex. 53 | See ex. 59 |
| $SiO_2/Al_2O_3$ (moles) | 28 | " | " | " | " | " | " | " |
| Amount of catalyst | (b) | " | " | " | " | " | " | " |
| Temp. (°C) | 597 | " | 550 | " | 500 | " | 450 | " |
| Space velocity (a) | 60 | " | 60 | " | 60 | " | 60 | " |
| Data survey after: | 1h | 5.30h | 1h | 4.30h | 1h | 4.30h | 1h | 6.15h |
| Results (% b.w.) Conversion | 92.4 | 91.6 | 91.2 | 85.5 | 92.0 | 88.6 | 91.6 | 90.0 |
| Selectivity to $C_3^-$ | 24.2 | 32.2 | 22.0 | 26.2 | 14.0 | 16.4 | 12.0 | 13.0 |
| Yield ($C_3^-$) | 22.4 | 29.5 | 20.1 | 22.4 | 12.9 | 14.5 | 11.0 | 11.7 |
| Sel. to $C_2^-$ | 6.74 | 8.46 | 4.95 | 2.96 | 2.35 | 1.84 | 1.64 | 1.33 |
| Sel. to saturated compounds $<C_4$ | 21.0 | 14.1 | 31.6 | 20.4 | 36.2 | 27.5 | 32.7 | 25.5 |
| Sel. to compounds $\geqslant C_5$ | 32.9 | 30.6 | 22.4 | 21.0 | 27.5 | 30.1 | 35.1 | 39.7 |
| Sel. to linear butenes | 15.2 | 14.6 | 19.0 | 29.4 | 20.0 | 24.2 | 18.7 | 20.5 |

(a) See ex. 1; (b) See ex. 4.

0 109 059

TABLE 4 (Continuation)

| Example | 61 | 62 | 63 | 64 | 65 | 66 | 67 | 68 |
|---|---|---|---|---|---|---|---|---|
| Operative Conditions: Catalyst | (*) H—ZSM5 | (*) See ex. 61 | See ex. 61 | See ex. 63 | See ex. 61 | See ex. 65 | See ex. 61 | See ex. 67 |
| SiO$_2$/Al$_2$O$_3$ (moles) | 28 | " | " | " | " | " | " | " |
| Amount of catalyst | (b) | " | " | " | " | " | " | " |
| Temp. (°C) | 550 | " | " | " | " | " | " | " |
| Space velocity (a) | 470 | " | 296 | " | 216 | " | 136 | " |
| Data survey after: | 1h | 5h | 1h | 4h | 1h | 5.15h | 1h | 6h |
| Results (% b.w.) Conversion | 76.2 | 69.6 | 85.2 | 81.8 | 86.8 | 81.0 | 88.2 | 85.2 |
| Selectivity to C$_3^-$ | 26.8 | 25.0 | 34.1 | 34.4 | 33.5 | 34.2 | 22.4 | 29.8 |
| Yield (C$_3^-$) | 20.4 | 17.4 | 29.1 | 28.1 | 29.1 | 27.7 | 19.8 | 25.4 |
| Sel. to C$_2^-$ | 1.61 | 1.30 | 4.92 | 3.81 | 5.49 | 3.48 | 2.85 | 3.64 |
| Sel. to saturated compounds <C$_4$ | 14.4 | 8.67 | 16.6 | 11.4 | 19.9 | 11.0 | 26.2 | 14.6 |
| Sel. to compounds ≥C$_5$ | 14.1 | 13.0 | 15.5 | 16.7 | 13.8 | 15.0 | 22.7 | 20.6 |
| Sel. to linear butenes | 43.1 | 52.0 | 28.8 | 33.7 | 27.4 | 36.2 | 25.8 | 31.4 |

(a) See ex. 1 (b) See ex. 4.
(*) Comparative examples: space velocity is too high and either conversion or selectivity to C$_3^-$ decrease with the time.

TABLE 4 (Continuation)

| Example | 69 | 70 | 71 | 72 | 73 | 74 |
|---|---|---|---|---|---|---|
| Operative Conditions: Catalyst | H—ZSM5 | See ex. 69 | H—ZSM5 | See ex. 71 | (*) H—ZSM5 | (*) See ex. 73 |
| $SiO_2/Al_2O_3$ (moles) | 28 | " | 204 | " | 28 | " |
| Amount of catalyst | (b) | " | | " | (b) | " |
| Temp. (°C) | 580 | " | 550 | " | 500 | " |
| Space velocity (a) | 216 | " | 60 | " | 60 | " |
| Data survey after: | 1h | 5.15h | 1h | 5.30h | 1h | 6.30h |
| Results (% b.w.) Conversion | 88.8 | 84.1 | 87.7 | 83.6 | 86.87 | 85.19 |
| Selectivity to $C_3^-$ | 39.3 | 39.9 | 42.7 | 44.8 | 31.96 | 31.97 |
| Yield ($C_3^-$) | 34.9 | 33.6 | 37.5 | 37.4 | 27.76 | 27.24 |
| Sel. to $C_2^-$ | 8.64 | 6.68 | 8.39 | 6.30 | 4.32 | 3.42 |
| Sel. to saturated compounds $<C_4$ | 16.0 | 10.0 | 17.4 | 10.7 | 19.31 | 14.60 |
| Sel. to compounds $\geqslant C_5$ | 15.0 | 15.4 | 10.2 | 10.4 | 20.31 | 23.33 |
| Sel. to linear butenes | 21.0 | 28.1 | 21.4 | 27.8 | 24.10 | 26.28 |

(a) See ex. 1; (b) See ex. 4.
(*) Zeolite H—ZSM5 was activated 2 hours at 700°C before being used.

TABLE 4 (Continuation)

| Example | 75 | 76 | 77 | 78 | 79 | 80 | 81 |
|---|---|---|---|---|---|---|---|
| Operative Conditions: Catalyst | Cr—H—ZSM5 | See ex. 75 | Mg—H—ZSM5 | See ex. 77 | See ex. 77 | (*) H—ZSM5 | (*) See ex. 80 |
| $SiO_2/Al_2O_3$ (moles) | 28 | " | See ex. 75 | " | " | 28 | " |
| Amount of catalyst | (b) | " | " | " | " | (b) | " |
| Temp. (°C) | 500 | " | " | " | " | 550 | " |
| Space velocity (a) | 60 | " | " | " | " | 3.5 | " |
| Data survey after: | 1h | 6h | 1h | 6.30h | 12h | 1h | 5.30h |
| Results (% b.w.) Conversion | 90.26 | 88.69 | 89.86 | 88.20 | 86.40 | 91.6 | 90.0 |
| Selectivity to $C_3^-$ | 25.44 | 29.49 | 26.66 | 29.65 | 32.65 | 12.0 | 13.0 |
| Yield $(C_3^-)$ | 22.96 | 26.15 | 23.98 | 26.15 | 28.21 | 11.0 | 11.7 |
| Sel. to $C_2^-$ | 4.84 | 4.83 | 4.83 | 4.32 | 4.01 | 4.01 | 3.42 |
| Sel. to saturated compounds $<C_4$ | 25.50 | 17.78 | 26.72 | 18.94 | 15.88 | 46.0 | 37.3 |
| Sel. to compounds $\geqslant C_5$ | 25.38 | 27.52 | 22.61 | 25.76 | 23.92 | 40.8 | 50.3 |
| Sel. to linear butenes | 18.83 | 20.58 | 19.18 | 21.34 | 23.54 | 3.0 | 3.0 |

(a) See ex. 1; (b) See ex. 4.
(*) Comparative examples.

# 0 109 059

## Claims

1. A process for converting olefins having from 4 to 12 carbon atoms in to propylene by contacting said olefins at a temperature of from 400 to 600°C with a ZSM5 or ZSM11 zeolite having a $SiO_2/Al_2O_3$ molar ratio $\leq 300$ which is used as such or in a modified form, optionally admixed with an inert binder, characterized in that the zeolite crystallites have an average size of $\leq 0.5$ µm and that the conversion is carried out at a space velocity higher than 50 kg/h of olefins per kg of pure zeolite (binder excluded).

2. The process of claim 1, wherein the zeolite has a molar ratio from 13 to 220, preferably from 25 to 220.

3. The process of claim 1 or 2, wherein the space velocity is from 60 to 300, preferably from 60 to $250h^{-1}$.

4. The process of any of claims 1 to 3, wherein the zeolite is activated in air, before being used, at a temperature of from 450 to 750°C, preferably 540 to 700°C.

5. The process of any of claims 1 to 4, wherein the zeolite is periodically regenerated with air at a temperature of from 400 to 600°C.

6. The process of any of claims 1 to 5, wherein the zeolite is admixed with a binder, preferably $SiO_2$ or $Al_2O_3$.

7. The process of any of claims 1 to 6, wherein the zeolite is used in its acid form.

8. The process of any of claims 1 to 7, wherein said olefins are selected from isobutene, n-butenes and mixtures of olefins having 5 to 8 carbon atoms.

9. The process of any of claims 1 to 8, wherein the zeolite is used in a modified form, the modifying element being selected from Mg, Ca, Sr, Ba, P, Cr, Cu, Zn and mixtures thereof.

10. A process according to any of claims 1 to 9 for converting mixtures of olefins having 4C atoms into propylene, said mixtures containing also $C_4^+$ paraffins which cannot be easily separated from olefins, said process comprising:

a) preliminary oligomerization of a $(C_4^- + C_4^+)$ mixture at a temperature of from 320 to 380°C in a catalyst bed of zeolitic nature, to obtain a mixture of olefins having from 5 to 8C atoms, the $C_4^+$ paraffins remaining unconverted;

b) cooling and condensation of the oligomerization effluent in order to separate the $C_4^+$ paraffins as a gaseous phase, and conversion of the remaining $(C_5^- - C_8^-)$ mixture to propylene under the operation conditions of claim 1;

c) cooling of the conversion effluent and compressing said effluent, preferably at 13 to 16 bar, whereby the hydrocarbons having 4 or more C atoms are condensed, the hydrocarbons having less than 4C atoms being separated as a gaseous phase.

## Patentansprüche

1. Verfahren zum Konvertieren von Olefinen mit 4 bis 12 Kohlenstoffatomen in Propylen durch Kontaktieren der Olefine bei einer Temperatur von 400 bis 600°C mit ZSM 5- oder ZSM 11- Zeolithen, die ein $SiO_2/Al_2O_3$-Molverhältnis $\leq 300$ aufweisen und als solche oder in modifizierter Form verwendet werden, gegebenenfalls im Gemisch mit einem inerten Bindemittel, dadurch gekennzeichnet, daß die Zeolith-Kristallite eine mittlere Größe von $\leq 0,5$ µm haben und die Konvertierung bei einer Raumgeschwindigkeit von mehr als 50 kg/h Olefine pro kg reinem Zeolith (Bindemittel ausgenommen) durchgeführt wird.

2. Verfahren nach Anspruch 1, worin der Zeolith ein Molverhältnis von 13 bis 220, vorzugsweise 25 bis 220, hat.

3. Verfahren nach Anspruch 1 oder 2, worin die Raumgeschwindigkeit 60 bis 300, vorzugsweise 60 bis 250 $h^{-1}$ beträgt.

4. Verfahren nach irgendeinem der Ansprüche 1 bis 3, worin der Zeolith vor der Verwendung an der Luft bei einer Temperatur von 450 bis 750°C, vorzugsweise 540 bis 700°C, aktiviert wird.

5. Verfahren nach irgendeinem der Ansprüche 1 bis 4, worin der Zeolith periodisch mit Luft bei einer Temperatur von 400 bis 600°C regeneriert wird.

6. Verfahren nach irgendeinem der Ansprüche 1 bis 5, worin der Zeolith mit einem Bindemittel, vorzugsweise $SiO_2$ oder $Al_2O_3$, vermischt ist.

7. Verfahren nach irgendeinem der Ansprüche 1 bis 6, worin der Zeolith in seiner sauren Form verwendet wird.

8. Verfahren nach irgendeinem der Ansprüche 1 bis 7, worin die Olefine ausgewählt sind unter Isobuten, n-Butenen und Mischungen von Olefinen mit 5 bis 8 Kohlenstoffatomen.

9. Verfahren nach irgendeinem der Ansprüche 1 bis 8, worin der Zeolith in modifizierter Form verwendet wird, wobei das modifizierende Element ausgewählt ist unter Mg, Ca, Sr, Ba, P, Cr, Cu, Zn und Mischungen davon.

10. Verfahren nach irgendeinem der Ansprüche 1 bis 9 zur Konvertierung von Mischungen von Olefinen mit 4 C-Atomen in Propylen, wobei die Mischungen auch $C_4^+$-Paraffine enthalten, die sich nicht leicht von Olefinen abtrennen lassen,

a) Einleitende Oligomerisation eines $(C_4^- + C_4^+)$-Gemisches bei einer Temperatur von 320 bis 380°C in

17

# 0 109 059

einem Katalysatorbett von zeolithischer Natur, um eine Mischung von Olefinen mit 5 bis 8 C-Atomen zu erhalten, wobei die $C_4^+$-Paraffine nicht konvertiert werden;

b) Abkühlen und Kondensieren des Oligomerisationsabstromes, um die $C_4^+$-Paraffine als Gasphase abzutrennen, und Konvertieren des zurückbleibenden $(C_5^- - C_8^-)$-Gemisches in Propylen unter den Arbeitsbedingungen von Anspruch 1;

c) Abkühlen des Konvertierungsabstromes und Komprimieren des Abstromes, vorzugsweise bei 13 bis 16 bar, wobei die Kohlenwasserstoffe mit 4 oder mehr C-Atomen kondensieren und die Kohlenwasserstoffe mit weniger als 4 C-Atomen als Gasphase abgetrennt werden.

## Revendications

1. Un procédé de transformation en propylène d'oléfines renfermant de 4 à 12 atomes de carbone par mise de ces oléfines au contact, à une température de 400 à 600°C, d'une zéolite ZSM-5 ou ZSM-11 dont le rapport molaire $SiO_2/Al_2O_3$ ≤300, utilisée telle quelle ou sous une forme modifiée, éventuellement mélangée avec un liant inerte, caractérisé en ce que les cristallites de zéolite ont une dimension moyenne ≤0,5 µm et en ce que l'on met la conversion en oeuvre à une vitesse spatiale supérieure à 50 kg/h d'oléfines par kg de zéolite pure (liant exclu).

2. Le procédé selon la revendication 1, dans lequel la zéolite présente un rapport molaire de 13 à 220, de préférence de 25 à 220.

3. Le procédé selon la revendication 1 ou 2, dans lequel la vitesse spatiale est comprise entre 60 et 300, de préférence entre 60 et 250 $h^{-1}$.

4. Le procédé selon l'une quelconque des revendications 1 à 3, dans lequel la zéolite est activée dans l'air avant son utilisation à une température de 450 à 750°C, de préférence de 540 à 700°C.

5. Le procédé selon l'une quelconque des revendications 1 à 4, dans lequel la zéolite est régénérée périodiquement avec de l'air, à une température de 400 à 600°C.

6. Le procédé selon l'une quelconque des revendications 1 à 5, dans lequel la zéolite est mélangée avec un liant, de préférence $SiO_2$ ou $Al_2O_3$.

7. Le procédé selon l'une quelconque des revendications 1 à 6, dans lequel la zéolite est utilisée sous sa forme acide.

8. Le procédé selon l'une quelconque des revendications 1 à 7, dans lequel ces oléfines sont choisies parmi l'isobutène, les n-butènes et les mélanges d'oléfines renfermant de 5 à 8 atomes de carbone.

9. Le procédé selon l'une quelconque des revendications 1 à 8, dans lequel la zéolite est utilisée sous une forme modifiée, l'élément de modification étant choisi parmi Mg, Ca, Sr, Ba, P, Cr, Cu, Zn et leurs mélanges.

10. Le procédé selon l'une quelconque des revendications 1 à 9, de conversion en propylène de mélanges d'oléfines renfermant 4 atomes de carbone, ces mélanges contenant également des paraffines en $C_4^+$ que l'on ne peut pas séparer facilement des oléfines, ce procédé consistant en:

a) une oligomérisation préliminaire d'un mélange $(C_4^- + C_4^+)$ à une température de 320 à 380°C dans un lit de catalyseur de nature zéolitique pour obtenir un mélange d'oléfines renfermant de 5 à 8 atomes de carbone, les paraffines en $C_4^+$ restant non transformées;

b) refroidissement et condensation de l'effluent de l'oligomérisation en vue de séparer les paraffines en $C_4^+$ sous la forme d'une phase gazeuse et conversion du mélange restant $(C_5^-$ à $C_8^-)$ dans les conditions opératoires de la revendication 1;

c) refroidissement de l'effluent de conversion et compression de cet effluent, de préférence à une pression de 13 à 16 bars, ce grâce à quoi les hydrocarbures renfermant 4 atomes de carbone ou plus sont condensés, les hydrocarbures renfermant moins de 4 atomes de carbone étant séparés sous la forme d'une phase gazeuse.

18

F I G U R E 1